# EUROPEAN PATENT APPLICATION

(11) **EP 4 825 963 A1**
(43) Date of publication of application: **21.10.2026**
(21) Application number: 24903707.8
(22) Date of filing: 11.12.2024
(51) Int. Cl.: C12N 9/88, C12N 1/15, C12N 1/19, C12N 1/21, C12N 5/10, C12N 9/12, C12N 15/10, C12N 15/54, C12N 15/60, C12N 15/63

(54) **RNA POLYMERASE VARIANT AND RNA SYNTHESIS**

(30) Priority: 12.12.2023 JP 2023209111; 31.01.2024 JP 2024013089
(71) Applicant: Takara Bio Inc., Kusatsu-shi Shiga 525-0058 (JP); Educational Corporation Kansai Bunri Sougougakuen, Shiga 526-0829 (JP)
(72) Inventor: MATSUMOTO, Hiroyuki, Kusatsu-shi, Shiga 525-0058 (JP); SAGARA, Takehiro, Kusatsu-shi, Shiga 525-0058 (JP); UEMORI, Takashi, Kusatsu-shi, Shiga 525-0058 (JP); YAMADA, Yukiho, Kusatsu-shi, Shiga 525-0058 (JP); SHIRAI, Tsuyoshi, Nagahama-shi, Shiga 526-0829 (JP)
(74) Representative: Müller, Christian Stefan Gerd
(86) International application number: PCT/JP2024/043752
(87) International publication number: WO 2025/127053

(57) **Abstract**

Provided is a variant of an RNA polymerase, the variant being characterized in that an amino acid corresponding to arginine at position 386 in the amino acid sequence of a wild-type T7 RNA polymerase represented by SEQ ID NO: 1 is substituted with an aromatic amino acid.

## Description

### TECHNICAL FIELD

The present invention relates to an RNA polymerase useful for production of single-stranded RNA, a nucleic acid encoding the RNA polymerase, and a method for producing a single-stranded RNA using the RNA polymerase.

### BACKGROUND ART

As a novel drug modality, mRNA drugs have attracted attention. The mRNA drug is a medicine containing, as an active ingredient, an artificially manufactured mRNA that encodes a protein. When the mRNA drug is administered to a living body, the protein is produced in the body to exert a medical effect based on the function of the protein. During the global SARS-CoV-2 infection outbreak in 2020, mRNA vaccines, a type of mRNA drugs, were put into practical use and administered to many people, significantly contributing to infection prevention. Advantages of mRNA drugs compared with other drugs include the design of vaccines in a short period of time based on viral genetic information, as well as the potential for mass production of different vaccines using a common manufacturing process, i.e., common equipment.

RNA is synthesized in living organisms through a transcription reaction catalyzed by RNA polymerase, using double-stranded DNA as a template. RNA transcription can be reconstituted ex vivo by providing necessary components for the reaction. However, many RNA polymerases found in prokaryotic and eukaryotic cells are composed of multiple subunits, and the need to produce these subunits separately has hindered practical application of RNA drugs. On the other hand, RNA polymerases derived from bacteriophages (T7 phage, T3 phage, K11 phage, etc.), chloroplasts, and mitochondria are composed of a single polypeptide (single-subunit DNA-dependent RNA polymerase: Non-Patent Document 1).

RNA polymerase is a useful tool in genetic engineering research. RNA polymerases derived mainly from T7 phage and SP6 phage have been commercially available as research reagents. Typical applications of RNA polymerase include the preparation of labeled RNA for use as hybridization probes, and nucleic acid amplification methods combined with reverse transcription and double-stranded cDNA synthesis. Since the basic enzymatic properties of bacteriophage-derived RNA polymerases had already been elucidated, these enzymes are now used in the industrial production of RNA.

In the industrial production of RNA, it is advantageous to use highly stable RNA polymerases. Therefore, variants of T7 RNA polymerase with improved thermal stability have been generated (Patent Document 1). Furthermore, attempts have been made to generate T7 RNA polymerase variants targeting E42, S43, Y44, E45, M46, G47, A255, R257, A258, G259, A260, L261, and A262 (Patent Document 2, Non-Patent Document 2).

On the other hand, when used in pharmaceuticals administered to humans, RNA needs to have a cap structure at its 5' end for efficient translation. Furthermore, it is known that double-stranded RNA (dsRNA) is produced as a byproduct in the transcription reaction by RNA polymerase. For example, double-stranded RNA administered into the human body can trigger therapeutically undesirable immune responses via Toll-like receptors and other pathways. Therefore, for the production of mRNA drugs composed of single-stranded RNA, a transcription reaction system that can suppress the generation of double-stranded RNA and synthesize RNA with a cap structure is advantageous.

### PRIOR ART DOCUMENT

### PATENT DOCUMENT

Patent Document 1: WO2001/066705
Patent Document 2: WO2019/036682

### NON-PATENT DOCUMENT

Non-patent Document 1: Biotechnology Journal International, 20(3), p1-35, 2017
Non-patent Document 2: NATURE BIOTECHNOLOGY VOLUME 41, p560-568, 2023

### SUMMARY OF THE INVENTION

### PROBLEMS TO BE SOLVED BY THE INVENTION

An object of the present invention is to provide an RNA polymerase variant that is more suitable for producing single-stranded RNA having a cap structure and has reduced double-stranded RNA production ability as compared with conventional RNA polymerase, for the purpose of stably supplying high-purity single-stranded RNA that can be used as a pharmaceutical or the like.

### SOLUTIONS TO THE PROBLEMS

As a result of diligent efforts to solve the above problems, the present inventors found an RNA polymerase variant that can efficiently transcribe RNA in the presence of a cap analog by introducing a novel mutation at a specific position in RNA polymerase. Furthermore, the present inventors constructed a high-purity single-stranded RNA synthesis system using the variant. Thus, the present invention was completed.

For example, the following embodiments are provided.
(1) An RNA polymerase variant wherein an amino acid corresponding to arginine at position 386 in the amino acid sequence of wild-type T7 RNA polymerase set forth in SEQ ID NO: 1 is substituted by an aromatic amino acid.
(2) The RNA polymerase variant according to (1), wherein the substitution mutation is introduced into an RNA polymerase comprising an amino acid sequence having 70% or more sequence identity with the amino acid sequence set forth in SEQ ID NO: 1.
(3) The RNA polymerase variant according to (1) or (2), which comprises an amino acid sequence having 90% or more sequence identity with the amino acid sequence set forth in SEQ ID NO: 1.
(4) The RNA polymerase variant according to any one of (1) to (3), wherein an amino acid corresponding to an amino acid selected from the group consisting of glutamic acid at position 48, arginine at position 50, phenylalanine at position 51, arginine at position 52, and proline at position 72 in the amino acid sequence set forth in SEQ ID NO: 1 is further substituted by an amino acid different from the original amino acid.
(5) The RNA polymerase variant according to (4), wherein an amino acid at a position corresponding to a position selected from the group consisting of positions 48, 50, 51, 52, and 72 in the amino acid sequence set forth in SEQ ID NO: 1 is substituted by alanine.
(6) The RNA polymerase variant according to any one of (1) to (5), wherein an amino acid corresponding to an amino acid selected from the group consisting of serine at position 430, serine at position 633, phenylalanine at position 849, and phenylalanine at position 880 in the amino acid sequence set forth in SEQ ID NO: 1 is further substituted by an amino acid different from the original amino acid.
(7) The RNA polymerase variant according to (6), wherein an amino acid corresponding to serine at position 430 in the amino acid sequence set forth in SEQ ID NO: 1 is substituted by proline, an amino acid corresponding to serine at position 633 in the amino acid sequence set forth in SEQ ID NO: 1 is substituted by proline, an amino acid corresponding to phenylalanine at position 849 in the amino acid sequence set forth in SEQ ID NO: 1 is substituted by isoleucine, or an amino acid corresponding to phenylalanine at position 880 in the amino acid sequence set forth in SEQ ID NO: 1 is substituted by tyrosine.
(8) A nucleic acid encoding the RNA polymerase variant according to any one of (1) to (7).
(9) A vector carrying the nucleic acid according to (8).
(10) A method for producing an RNA polymerase variant, the method comprising a step of culturing a cell into which the nucleic acid according to (8) has been introduced.
(11) A method for producing a single-stranded RNA, the method comprising using a reaction solution containing the RNA polymerase variant according to any one of (1) to (7).
(12) The method according to (11), wherein the reaction solution further contains a single-stranded DNA-binding protein, a single-stranded RNA-binding protein, or a cold shock protein.
(13) The method according to (12), wherein the single-stranded DNA-binding protein is a T4 phage gene 32 protein.
(14) The method according to (12), wherein the reaction solution contains a cold shock protein.
(15) The method according to (14), wherein the cold shock protein is CspA.

### EFFECTS OF THE INVENTION

According to the present invention, an RNA polymerase variant having RNA transcription activity suitable for the production of high-purity RNA in vitro, and a method for producing the RNA polymerase variant are provided. According to the present invention, an RNA polymerase into which a mutation has been introduced at a specific amino acid position can synthesize single-stranded RNA more efficiently in a reaction solution containing a cap analog, while the production of double-stranded RNA is suppressed. Therefore, the RNA polymerase variant of the present invention exhibits excellent effects in supplying RNA with higher purity than conventional RNA polymerases.

### BRIEF DESCRIPTION OF THE DRAWINGS

[Fig. 1] Figure 1 shows the amount of total RNA synthesized using the RNA polymerase variant of the present invention in the presence or absence of a cap analog.
[Fig. 2] Figure 2 shows the amount of protein expressed from RNA synthesized using the RNA polymerase variant of the present invention.
[Fig. 3] Figure 3 shows the dsRNA contents in RNAs of different chain lengths synthesized using the RNA polymerase variant of the present invention in the presence of a cap analog.
[Fig. 4] Figure 4 shows the cap retention rate of RNA synthesized using the RNA polymerase variant of the present invention.

### MODE FOR CARRYING OUT THE INVENTION

### (Definition of Terms)

As used herein, nonpolar aliphatic amino acids refer to glycine, alanine, leucine, isoleucine, valine, and proline, which may be denoted by G, A, L, I, V, and P, respectively. Polar amino acids refer to serine, threonine, glutamine, and asparagine, which may be denoted by S, T, Q, and N, respectively. Acidic amino acids refer to glutamic acid and aspartic acid, which may be denoted by E and D, respectively. Basic amino acids refer to lysine, arginine, and histidine, which may be denoted by K, R, and H, respectively. Aromatic amino acids refer to phenylalanine, tyrosine, and tryptophan, which may be denoted by F, Y, and W, respectively. Sulfur-containing amino acids refer to cysteine and methionine, which may be denoted by C and M, respectively.

As used herein, amino acid sequence identity refers to the percentage of the number of amino acid residues that are identical between two amino acid sequences optimally aligned with each other. When a position in the first sequence is occupied by the same amino acid residue as the corresponding position in the second sequence, the sequences have identity with respect to that residue position. Proper alignment of two sequences can be performed using various algorithms, for example the BLAST algorithm.

As used herein, amino acid mutation includes deletion, addition, insertion, or substitution of one or more amino acids.

Hereinafter, the present invention is explained in detail.

### 1. RNA polymerase variant of the present invention

The present invention provides an RNA polymerase variant that is suitable for producing single-stranded RNA used in mRNA drugs and the like.

RNA polymerase is an enzyme having activity for synthesizing RNA chains complementary to the sequence of DNA as a template. Among RNA polymerases, an RNA polymerase consisting of a single polypeptide chain is referred to as single-subunit DNA-dependent RNA polymerase. The RNA polymerase variant of the present invention is obtained by introducing a mutation into an amino acid sequence of this type of RNA polymerase. While not particularly limited, the RNA polymerase variant provided by the present invention exhibits improved transcription efficiency in a reaction solution containing a cap analog.

The RNA polymerase variant of the present invention (hereinafter sometimes referred to as "the variant of the present invention") is preferably a variant of a bacteriophage-derived RNA polymerase, and examples thereof include a variant of a T7 phage RNA polymerase, and a variant of an RNA polymerase having high amino acid sequence identity with the T7 phage RNA polymerase (for example, an RNA polymerase derived from the T3 phage, K11 phage, or SP6 phage). Examples of the RNA polymerase variant of the present invention include, but not limited to, variants of RNA polymerases comprising an amino acid sequence having 70% or more sequence identity with the amino acid sequence of wild-type T7 RNA polymerase set forth in SEQ ID NO: 1. The amino acid sequences of T3 phage RNA polymerase (NCBI ACCESSION _CAC86264) and K11 phage RNA polymerase (NCBI ACCESSION_P18147) share more than 70% identity with the amino acid sequence of T7 RNA polymerase. Therefore, it is easy to identify amino acids in T3 polymerase and K11 polymerase, corresponding to a specific amino acid present in the amino acid sequence of wild-type T7 RNA polymerase set forth in SEQ ID NO: 1 (see Non-Patent Document 1). For example, position 387 in T3 RNA polymerase and position 409 in K11 RNA polymerase correspond to R386 in T7 RNA polymerase. An aspect of the present invention provides a variant polypeptide having 90% or more, preferably 95% or more, and more preferably 98% or more sequence identity with the amino acid sequence of the T7 RNA polymerase set forth in SEQ ID NO: 1. The variant polypeptide has RNA polymerase activity, that is, the ability to transcribe RNA from DNA as a template.

Hereinafter, the present invention will be explained with reference to a variant derived from T7 RNA polymerase as an example. A variant of the wild-type T7 RNA polymerase set forth in SEQ ID NO: 1, in which R386 is substituted by an aromatic amino acid, synthesizes a single-stranded RNA having a cap structure in a reaction solution containing a cap analog with higher efficiency than the wild-type T7 RNA polymerase. In the variant of the present invention, R386 is preferably substituted by phenylalanine, tyrosine, or tryptophan.

The RNA polymerase variant of the present invention may further comprise mutation, for example substitution, of one or more amino acids, in addition to the substitution of an amino acid corresponding to R386 in the amino acid sequence of wild-type T7 RNA polymerase. This additional mutation is not particularly limited as long as the mutation does not result in a significant decrease in the RNA transcription activity of the variant of the present invention. Such an additional substitution may be, for example, substitution of an amino acid corresponding to an amino acid selected from E48, R50, F51, R52, or P72 in T7 RNA polymerase. Substitution of one or more of the five amino acids by other amino acids can reduce the ability of the variant of the present invention to produce double-stranded RNA. The amino acid after substitution is not particularly limited, but is preferably a nonpolar aliphatic amino acid, more preferably alanine.

Furthermore, the RNA polymerase variant of the present invention may comprise a known mutation. Patent Document 1 discloses an RNA polymerase variant with improved heat resistance, obtained by introducing four amino acid substitutions (serine at position 430, serine at position 633, phenylalanine at position 849, and phenylalanine at position 880 are substituted by proline, proline, isoleucine, and tyrosine, respectively) into T7 RNA polymerase which is a single-subunit DNA-dependent RNA polymerase (the amino acid sequence of wild-type T7 RNA polymerase is set forth in SEQ ID NO: 1). Into the RNA polymerase variant of the present invention, substitution of one or more amino acids corresponding to an amino acid selected from the above-mentioned amino acid S430, S633, F849, or F880 may be introduced. One or more mutations can be introduced into the RNA polymerase variant of the present invention, wherein the one or more mutations are preferably selected from substitution of an amino acid corresponding to S430 by proline, substitution of an amino acid corresponding to S633 by proline, substitution of an amino acid corresponding to F849 by isoleucine, or substitution of an amino acid corresponding to F880 by tyrosine.

A variant of T7 RNA polymerase in which R386 is substituted by tryptophan, and in addition, S430, S633, F849, and F880 are substituted by proline, proline, isoleucine, and tyrosine, respectively, is named T7-a2-a27. The amino acid sequence of T7-a2-a27 is shown in SEQ ID NO: 6. In other words, a polypeptide comprising the amino acid sequence set forth in SEQ ID NO: 6 is an example of the variant of the present invention. T7-a2-a27 exhibits improved RNA transcription efficiency compared with the wild-type T7 RNA polymerase, particularly in the presence of a cap analog. Furthermore, variants of T7-a2-a27 in which R50 or F51 is substituted by alanine are named T7-b7 and T7-b8, respectively. These two variants are examples of the variant of the present invention, exhibiting significantly reduced double-stranded RNA producing ability compared with the wild-type T7 RNA polymerase.

In one aspect of the present invention, other peptides may be added to the variant of the present invention. Examples of such other peptides include, but not limited to, signal peptides necessary for the secretory expression of the variant of the present invention, and affinity tags useful for the purification of the variant of the present invention. The signal peptide may be selected from suitable peptides derived from known secretory proteins, or modified peptides thereof, depending on a host used to produce the variant of the present invention. The signal peptide is usually attached to the N-terminus of the variant of the present invention. The affinity tag may be any affinity tag for which a ligand capable of capturing the affinity tag is known, and may be selected from known affinity tags. Examples of the affinity tag include histidine (His) tags, HN tags, HAT tags, glutathione S-transferase (GST) tags, maltose-binding protein (MBP) tags, C-Myc tags, and the Strep(II) tag, which consists of eight amino acid residues (Trp-Ser-His-Pro-Gln-Phe-Glu-Lys). These tags may be attached to either the N-terminus or the C-terminus of the variant of the present invention. Furthermore, the affinity tag can be removed from the variant of the present invention after the purification process is completed. For this purpose, an affinity tag is used that allows insertion of a recognition sequence for protease, such as Factor Xa, PreScission Protease, thrombin, enterokinase, or TEV protease (Tobacco Etch Virus Protein), between the RNA polymerase variant of the present invention and the affinity tag.

The RNA polymerase variant of the present invention can be prepared using known methods for preparing recombinant proteins. Based on the nucleotide sequence information of a gene encoding a wild-type single-subunit DNA-dependent RNA polymerase, a codon encoding an amino acid corresponding to R386 in the amino acid sequence of T7 RNA polymerase may be identified and changed to a codon for an aromatic amino acid. A nucleic acid having the nucleotide sequence thus designed, i.e., a nucleic acid encoding the variant of the present invention, can be prepared and used for production of the variant of the present invention using a suitable expression vector and host to obtain as described below. For introduction of a mutation at a position other than the position corresponding to R386, a nucleotide sequence encoding a desired variant may be designed using the same procedure. The nucleic acid encoding the variant of the present invention may be chemically synthesized, or may be prepared by introducing a site-directed mutation into a nucleic acid encoding a wild-type RNA polymerase.

The RNA polymerase variant of the present invention can be used not only for the production of RNA, but also for nucleic acid amplification methods that include RNA polymerase as a component, (for example, the NASBA method described in U.S. Patent No. 5130238: Nucleic Acid Sequence-based Amplification, the 3SR method described in WO90/06995: Self-sustained sequence replication reaction, the SMART method described in WO99/37806: Signal mediated amplification of RNA technology, the TMA method described in WO91/01384: Translation-mediated amplification, etc.). These methods can efficiently amplify a target nucleic acid under isothermal conditions.

### 2. Nucleic acid encoding RNA polymerase variant of the present invention

The present invention provides a nucleic acid encoding the RNA polymerase variant described in 1. (hereinafter, sometimes referred to as "the nucleic acid of the present invention").

The nucleic acid of the present invention may be any nucleic acid that encodes the RNA polymerase variant of the present invention, and is not limited to a nucleic acid of a specific nucleotide sequence. As described above, the nucleotide sequence of the nucleic acid of the present invention is designed by identifying a codon corresponding to an amino acid to be substituted based on the nucleotide sequence information of a gene encoding a wild-type RNA polymerase, and then changing the identified codon to a codon for another amino acid. In order to increase the expression level of the RNA polymerase variant of the present invention in a host, the designed codon nucleotide sequence may be changed to a nucleotide sequence suited for the host without changing the amino acid sequence encoded by the sequence (codon optimization).

As described above, a signal peptide or affinity tag may be attached to the RNA polymerase variant of the present invention. Therefore, the nucleic acid of the present invention includes a nucleic acid encoding the variant of the present invention to which a signal peptide or affinity tag is attached. The nucleotide sequence of a nucleic acid encoding a signal peptide or affinity tag may be designed according to its amino acid sequence, and codon selection may be performed considering a host used.

The nucleic acid encoding the RNA polymerase variant of the present invention may be chemically synthesized, or may be prepared by introducing a site-directed mutation into a nucleic acid encoding a wild-type RNA polymerase. A method for introducing a site-directed mutation into a nucleic acid is well known to those skilled in the art, and a kit for use in the method is commercially available.

The PET system, a recombinant protein expression system using *Escherichia coli* as a host, utilizes a bacteriophage-derived RNA polymerase. The expression of a desired protein is strongly induced by introducing a nucleic acid encoding the desired protein placed downstream of a promoter recognized by RNA polymerase into *Escherichia coli* and expressing the RNA polymerase within the same *Escherichia coli.* In this system, the RNA polymerase is supplied either from an RNA polymerase gene integrated into *Escherichia coli* chromosomes or from an expression vector introduced into *Escherichia coli.* The nucleic acid of the present invention can be used as a source of RNA polymerase in PET systems and similar expression systems.

### 3. Vector comprising nucleic acid encoding the RNA polymerase variant of the present invention.

The present invention provides a vector carrying a nucleic acid encoding the RNA polymerase variant of the present invention. The vector of the present invention is useful for introducing a nucleic acid encoding the RNA polymerase variant of the present invention into a suitable host for the production of the RNA polymerase variant of the present invention and other purposes.

A vector into which the nucleic acid encoding the RNA polymerase variant of the present invention is inserted is not particularly limited. A vector that can autonomously replicate in a host cell or a vector that can be incorporated into a chromosome of a host can be used. For example, a plasmid vector, a phage vector, a viral vector, an artificial chromosome, etc., can be used. A vector suitable for the host used is selected. Vectors suitable for various hosts *(Escherichia coli,* Bacillus bacteria, yeast, filamentous fungi, insect cells, mammalian cells, etc.) are well known to those skilled in the art, and many of such vectors are commercially available. Such known vectors or modified vectors therefrom can be utilized in the present invention. Numerous expression vectors have been constructed to express recombinant proteins in hosts.

The expression vector contains an appropriate promoter and other factors involved in transcription and translation (an operator, a terminator, an enhancer, a ribosome-binding site, etc.) that can function in a host, and is therefore suitable for generation of the product of a gene carried by the vector in the host. Examples of an expression vector used for *Escherichia coli* include, but not limited to, a trp promoter, a lac promoter, a PL promoter, and a PR promoter, and modified promoters thereof. When an inducible promoter is used, the expression of the gene product can be induced through appropriate manipulation.

The expression vector used in the present invention may further contain a nucleic acid encoding a signal peptide or affinity tag. The nucleic acids encoding these peptides are arranged to be expressed as a fusion protein of the RNA polymerase variant of the present invention and the affinity tag. Vectors into which a nucleic acid encoding an affinity tag has been inserted are also known. The nucleic acid of the present invention may be inserted into such a vector to prepare the vector of the present invention. The expression vector containing the nucleic acid of the present invention thus constructed is useful in production of the RNA polymerase variants of the present invention.

### 4. Method for producing RNA polymerase variant of the present invention

The present invention provides a method for producing the RNA polymerase variant of the present invention. The method includes a step of culturing a cell into which a nucleic acid encoding the variant of the present invention has been introduced, and is carried out by collecting the RNA polymerase variant from the resulting culture.

A cell (host) used for production of the variant of the present invention may be any host used in production of recombinant proteins, and is not particularly limited. Examples of the host that can be used include bacteria (for example, *Escherichia coli, Bacillus subtilis,* etc.), yeast, filamentous fungi, insect cells, eukaryotic cells, and animal cells (for example, mammalian cells including human cells).

Typical hosts used in industrial production of recombinant proteins, such as *Escherichia coli,* and Bacillus species including *Bacillus subtilis* are well known to those skilled in the art, and many strains are commercially available. From among these, a suitable host strain can be selected and used, taking into consideration the manufacturing scale and other conditions. Furthermore, methods for producing recombinant proteins, comprising using yeast (such as genus Saccharomyces, and genus Schizosaccharomyces) or an insect cell as a host are also known. The RNA polymerase variant can be expressed and produced by introducing a nucleic acid encoding the RNA polymerase variant of the present invention into the host cell, or by preparing a host cell in which the nucleic acid has been integrated into the chromosome, and then culturing the host cell.

The nucleic acid encoding the RNA polymerase variant of the present invention can be loaded onto a suitable vector and then introduced into a host. As the vector, for example, a vector that can be used as an expression vector may be selected. When using a plasmid vector, a method for introducing the vector can be appropriately selected depending on the host. Examples of the method include methods comprising using calcium ions, lipofection, electroporation, spheroplasts, and lithium acetate. Phage vectors and viral vectors infect host cells in a manner suitable for each vector, whereby cells into which the nucleic acid encoding the RNA polymerase variant has been introduced can be obtained. Furthermore, the PET system may be used in the production of the variant of the present invention.

The cell containing the nucleic acid encoding the RNA polymerase variant thus obtained is cultured, and the variant of the present invention can be obtained from the culture. There are no particular limitations on the culture conditions, as long as they are suitable for the host, expression vector, etc. used. For separation or purification of the RNA polymerase variant, known protein purification methods can be used. Hereinafter, the case where *Escherichia coli* is used as a host will be explained. *Escherichia coli* cells into which the nucleic acid encoding the variant of the present invention has been introduced are cultured at a temperature suitable for the growth of *Escherichia coli,* for example, 37°C. When the nucleic acid encoding the RNA polymerase variant is located downstream of an inducible promoter, the expression of the RNA polymerase variant can be induced by performing a procedure appropriate to the promoter during culture. The *Escherichia coli* cells collected from the culture and washed can be subjected to ultrasonic disruption, lysozyme treatment, or other lysis treatment to obtain an *Escherichia coli* lysate containing the variant of the present invention. When the variant of the present invention is expressed and secreted, the culture supernatant is collected. Using the lysate or the culture supernatant as a starting material, the RNA polymerase variant of the present invention is purified by appropriately combining purification methods used in the art, for example, ammonium sulfate precipitation, anion exchange column, cation exchange column, gel filtration column, affinity chromatography column, filtration, dialysis, etc. The variant to which an affinity tag has been attached can be readily purified using an affinity carrier suitable for the properties of the affinity tag. For example, the variant of the present invention having an affinity tag containing multiple histidine residues (histidine tags, HN tags, HAT tags, etc.) can be purified using a carrier bound to a metal such as nickel.

### 5. Method for producing single-stranded RNA of the present invention

The present invention provides a method for producing a single-stranded RNA (in vitro transcription method), comprising using the RNA polymerase variant of the present invention. In the presence of a double-stranded DNA as a template and four types of ribonucleotide triphosphates, the variant of the present invention synthesizes a single-stranded RNA comprising a sequence complementary to the template DNA. Examples of the RNA produced by the method of the present invention include, but not limited to, mRNA, RNA probes, ribozymes, and guide RNA (used for genome editing with Cas9, etc.).

The double-stranded DNA that can be used as a template in the method for producing a single-stranded RNA of the present invention has a promoter sequence recognized by the variant of the present invention. Promoter sequences recognized by bacteriophage-derived RNA polymerases are known (see, for example, FEBS Letters, 4, pp. 264-267, 1998). Furthermore, the 3' end of the region encoding the RNA to be transcribed may comprise a sequence encoding a poly(A) tail. The origin of the template double-stranded DNA is not particularly limited, and the template double-stranded DNA may be a plasmid DNA, or a DNA fragment obtained by nucleic acid amplification. A double-stranded DNA that can be used as a template can be prepared, for example, by inserting a DNA comprising a nucleotide sequence encoding a desired RNA downstream of a promoter recognized by RNA polymerase into a plasmid containing the promoter. A circular DNA such as a plasmid is preferably used after linearization (e.g., digestion with restriction enzymes).

Four ribonucleotide triphosphates (ATP, CTP, GTP, and UTP) are typically used as substrates for native RNA, and however, one or more of the four ribonucleotide triphosphates may be replaced with ribonucleotide triphosphate analogs. The ribonucleotide triphosphate analogs are not particularly limited. Known ribonucleotide triphosphate analogs known as substrates for RNA polymerase can be used in the method of the present invention. For example, in production of RNA to be administered to living organisms, pseudouridine triphosphate, 1-methylpseudridine triphosphate, or other UTP analogs may be used instead of UTP. The concentrations of ribonucleotide triphosphates or their analogs can be appropriately determined depending on the purpose, and is usually in the range of 0.2 to 15 mM.

A reaction solution used for in vitro transcription contains a buffering component to maintain an appropriate pH level, as well as a magnesium ion and a reducing agent (for example, dithiothreitol). The reaction solution may also contain other various components, for example, a salt (NaCl, KCl, etc.), polyamine (spermidine, etc.), a ribonuclease inhibitor, protein (bovine serum albumin, etc.), a surfactant, etc. The concentrations of these components can be determined with reference to the compositions of known reaction solutions. The pH of the reaction solution is usually adjusted to 7.0-9.0.

Eukaryotic mRNA has a modification called a cap structure at its 5' end. The modification consists of 7-methylguanosine linked to the 5' end via a 5'-5' triphosphate structure, and methylation at the 2'-O position of a nucleotide adjacent to the 7-methylguanosine. The cap structure contributes to stabilization of mRNA, and further, improves translation efficiency by being recognized by translation initiation factors in eukaryotic cells.

A cap structure can be added to a single-stranded RNA produced by the method of the present invention. When a single-stranded RNA is treated with a capping enzyme, for example a capping enzyme derived from vaccinia virus, 7-methylguanosine is added to the 5' end of the single-stranded RNA (Cap0). Subsequently, reaction with a mRNA Cap 2'-O-methyltransferase results in methylation at the 2'-O position of a nucleotide adjacent to the 7-methylguanosine (Cap1).

In the method of the present invention, addition of a cap structure can be performed concurrently with transcription of a single-stranded RNA. For this purpose, the transcription of a single-stranded RNA using the variant of the present invention is performed in a reaction solution containing a compound called a cap analog. The cap analog is not particularly limited, and may be any cap analog composed of dinucleotides, trinucleotides, or more nucleotides. For example, commercially available cap analogs such as m7G(5')ppp(5')G (standard cap), ARCA (Anti-Reverse Cap Analog), and CleanCap (registered trademark) series cap analogs (manufactured by TriLink) can also be used in the present invention.

The reaction solution of the present invention may contain an inorganic pyrophosphatase. When RNA is synthesized by RNA polymerase, pyrophosphate accumulates as a byproduct, and pyrophosphate inhibits transcription. Inorganic pyrophosphatase has activity to catalyze the hydrolysis of pyrophosphate to produce orthophosphate. Therefore, the breakdown of pyrophosphate by inorganic pyrophosphatase promotes RNA synthesis reaction.

Furthermore, the present invention provides a method for reducing the generation of a double-stranded RNA in an RNA transcription reaction, the method comprising using a reaction solution containing a protein having affinity for nucleic acids. Examples of the protein having affinity for nucleic acids include a single-stranded DNA-binding protein (SSB), a single-stranded RNA-binding protein, and a cold shock protein. When a reaction solution for RNA transcription containing a protein having affinity for nucleic acids is used, the generation of double-stranded RNA is suppressed compared with when a reaction solution not containing the protein is used. Examples of the single-stranded DNA-binding protein include a T4 phage gene 32 protein (T4 gp32), a T7 phage single-stranded DNA-binding protein, and a single-stranded DNA-binding protein of *Escherichia coli.* Cold shock protein is a general term for proteins that are transiently expressed at high levels in bacteria and the like when their growth temperature decreases. In *Escherichia coli,* a cold shock protein named CspA is known. Furthermore, eight types of proteins: CspB to CspI are known to have high amino acid sequence identity with CspA. Of these, CspB, CspG and CspI are cold shock proteins [J. Bacteriol., 181, pp. 1603-1609, 1999]. Furthermore, homologues of these cold shock proteins are present in microorganisms such as *Bacillus subtilis* (CspB), *Bacillus caldolyticus* (CspB), *Thermotoga maritima* (CspB, CspL), and *Lactobacillus plantarum* (CspL). While the present invention is not particularly limited, CspA from *Escherichia coli* is preferably used for RNA transcription reaction. For the method for reducing the generation of double-stranded RNA in an RNA transcription reaction, any RNA polymerase may be used in the RNA transcription reaction, and a wild-type RNA polymerase or a variant RNA polymerase may be used. The variant RNA polymerase is also not limited to the RNA polymerase variant of the present invention. Furthermore, a reaction solution containing the protein having affinity for nucleic acids can be also used in the method for producing a single-stranded RNA of the present invention.

### 6. Kit of the present invention

The present invention provides a kit containing the RNA polymerase variant of the present invention for use in synthesis of a desired RNA.

The kit of the present invention may contain, in addition to the variant of the present invention, components necessary for preparing a reaction solution for RNA transcription, for example a buffering component, a magnesium salt, four types of NTPs or NTP analogs, polyamine, a reducing agent, a surfactant, and other components. These components may be packaged individually so that they can be mixed at the time of use to prepare the reaction solution, or may be packaged as a mixture of multiple components to facilitate the preparation of the reaction solution. Examples of the kit include a kit containing a premix solution prepared so that the reaction solution is completed by adding only a template DNA and water (sterilized water, etc.) at the time of use, and a kit containing the premix in a dry state. Other components that may be contained in the kit include a ribonuclease inhibitor, a cap analog, an inorganic pyrophosphatase, bovine serum albumin, and other proteins (for example, a single-stranded DNA-binding protein, a single-stranded RNA-binding protein, a cold shock protein, etc.).

### EXAMPLES

Hereinafter, the present invention will be explained in detail with reference to Examples to which the scope of the present invention is not limited.

### Experimental Method

### (1) Method for preparing T7 RNA polymerase variant

The nucleotide sequence of a gene encoding a wild-type RNA polymerase from Escherichia phage T7 (Bacteriophage T7) strain is disclosed in NCBI Reference Sequence No. [NC_001604 GeneID: 1261050]. The amino acid sequence of the wild-type T7 RNA polymerase encoded by the above-mentioned nucleotide sequence is set forth in SEQ ID NO: 1 in the sequence listing. An artificial gene having a nucleotide sequence encoding an amino acid sequence into which a desired mutation was introduced into the above-mentioned amino acid sequence was chemically synthesized. The obtained artificial gene was introduced into plasmid pET6xHN-N (manufactured by Takara Bio USA) using In-Fusion (registered trademark) HD Cloning Kit (manufactured by Takara Bio USA). The resulting plasmid had a nucleotide sequence encoding a T7 RNA polymerase variant with a histidine tag attached to its N-terminus.

Next, *Escherichia coli* strain BL21 DE3 (manufactured by Takara Bio Inc.) was transformed with the plasmid, and incubated overnight at 37°C on a 1.5% agarose LB plate containing 100 µg/mL ampicillin. A single colony from this plate was inoculated into LB medium containing 100 µg/mL of ampicillin (hereinafter referred to as LB-AP medium), and cultured at 37°C overnight with shaking. This culture medium (300 µl) was inoculated into 25 mL of LB-AP medium, and cultured at 37°C overnight with shaking. When the OD600 value reached 0.6, IPTG was added to the culture medium at a final concentration of 1 mM, followed by incubation at 30°C for 4 hours for induction, after which the bacterial cells were harvested.

The bacterial cells obtained above were suspended in 2 mL of a solution containing 50 mM Tris·HCl pH 8.0, 100 mM NaCl, 1 mM EDTA (pH 8.0), and 5% glycerol. Lysozyme (manufactured by Sinopharm Chemical Reagent Co., Ltd.) was added to the suspension at a final concentration of 0.1 mg/mL, followed by shaking at 4°C for 1 hour. After shaking, the mixture was centrifuged at 4°C and 15,000 x g for 30 minutes, and a supernatant was collected. The supernatant was applied to a Ni-NTA Agarose (Qiagen) column to adsorb the RNA polymerase variant. After washing the column, the adsorbed material was eluted with a buffer solution containing imidazole. Next, the obtained eluate was applied to a POROS HQ (Thermo Fisher Scientific) column to adsorb the RNA polymerase variant. After washing the column, the adsorbed material was eluted with a buffer solution containing sodium chloride. The obtained eluate was dialyzed, and the buffer solution was replaced by a solution containing 20 mM potassium phosphate (pH 7.9), 100 mM NaCl, 0.1 mM EDTA, 1 mM DTT, and 50% glycerol. The resulting buffer-replaced solution was used as an RNA polymerase variant solution in each experiment.

The polymerase activity in the RNA polymerase variant solution was measured using a reaction mixture with composition as below. The RNA polymerase activity (number of units) in the solution was calculated by defining 1 U as the amount of enzyme that incorporates 1 nmol of [³H]GMP into an acid-insoluble precipitate per hour at 37°C.

### [Composition of reaction solution (50 µl) for activity measurement]

| | |
|---|---|
| 40 mM | Tris-HCl (pH 8.0) |
| 8 mM | MgCl₂ |
| 2mM | Spermidine |
| 5mM | DTT |
| 0.4mM | ATP, UTP, CTP |
| 0.4mM | [³H]GTP |
| 1µg/50µl | pT7-2 DNA (manufactured by USB) |

### (2) Method for evaluating amount of transcribed RNA and amount of produced dsRNA

Using the prepared T7 RNA polymerase variant, RNA transcription reaction was tested by the following method. Using 10X T7 RNA Polymerase Buffer included with T7 RNA Polymerase ver2.0 (product number 2541, manufactured by Takara Bio Inc.), a final volume of 20 µL of a reaction solution containing 10 mM NTP at the final concentration, 1 µg of Positive Control Template (FLuc) (a linear DNA comprising a sequence encoding firefly luciferase located downstream of a T7 promoter) included with Takara IVTpro^{™} (trademark) mRNA Synthesis System (product number 6141, manufactured by Takara Bio Inc.) as a template DNA, 0.1 U of pyrophosphatase (inorganic) (product number 2450, manufactured by Takara Bio Inc.), 20 U of RNase Inhibitor ver. 2.0 (product number 2315, manufactured by Takara Bio Inc.), and 200 U of the T7 RNA polymerase variant prepared in Experimental Method (1) was prepared. As a control, a reaction solution containing the wild-type T7 RNA polymerase was also prepared.

The RNA transcription reaction was carried out at 37°C for 120 minutes using ThermalCycler TP-990 ThermalCycle Dice (registered trademark) Real Time System III (manufactured by Takara Bio Inc.). Subsequently, the reaction solution was treated with 10 U equivalent of DNase I (product number 2270, manufactured by Takara Bio Inc.) at 37°C for 15 minutes to degrade the template DNA. Then, the reaction solution was placed on ice to stop the reaction.

The reaction solution treated with DNase I was subjected to precipitation and purification with lithium chloride at a final concentration of 3M, and the resulting precipitates were finally dissolved in RNase-free water. The RNA concentration in the RNA solution thus obtained was determined using a spectrophotometer (1 OD at 260 nM = 40 µg/ml RNA). The amount of RNA in the reaction solution calculated from the RNA concentration thus obtained represents the sum of single-stranded RNA and double-stranded RNA (total RNA amount). The amount of double-stranded RNA was determined using EasyAna dsRNA (Modified) Quantitative Detection Kit (ELISA) 2.0 (product number DD3509EN, manufactured by Vazyme), following the instructions attached to the kit.

### Example 1: Preparation of T7 RNA polymerase variant

Artificial genes encoding variant proteins in which the amino acid substitutions shown in Table 1 were introduced into the amino acid sequence of wild-type T7 RNA polymerase were designed and chemically synthesized using known methods. According to Experimental Method (1), recombinant plasmids containing the obtained artificial genes were prepared, and the variants were expressed and purified. Table 1 shows the names and SEQ ID NOs (SEQ ID NOs: 2-17) of the amino acid sequences of the T7 RNA polymerase variants into which each mutation was introduced.

**[Table 1]**

| SEQ ID NO: | Name | Mutation position in prepared variant | Mutation added to T7-a2_a27 |
|---|---|---|---|
| 1 | T7-WT | None | |
| 2 | a27 | R386W | |
| 3 | a28 | R386F | |
| 4 | a29 | R386Y | |
| 5 | T7-a2 | S430P, S633P, F849I, F880Y | |
| 6 | T7-a2_a27 | S430P, S633P, F8491, F880Y, R386W | |
| 7 | T7-b2 | S430P, S633P, F8491, F880Y, R386W, L37A | L37A |
| 8 | T7-b3 | S430P, S633P, F849I, F880Y, R386W, L39A | L39A |
| 9 | T7-b4 | S430P, S633P, F8491, F880Y, R386W, E40A | E40A |
| 10 | T7-b5 | S430P, S633P ,F8491, F880Y, R386W, H41A | H41A |
| 11 | T7-a2_a5_a27 | S430P, S633P, F849I, F880Y, R386W, SY43-44AA | SY43-44AA |
| 12 | T7-b1 | S430P, S633P, F8491, F880Y, R386W, Y44A | Y44A |
| 13 | T7-b6 | S430P, S633P, F849I, F880Y, R386W, E48A | E48A |
| 14 | T7-b7 | S430P, S633P, F849I, F880Y, R386W, R50A | R50A |
| 15 | T7-b8 | S430P, S633P, F8491, F880Y, R386W, F51A | F51A |
| 16 | T7-b9 | S430P, S633P, F849I, F880Y, R386W, R52A | R52A |
| 17 | T7-a2_a7_a27 | S430P, S633P, F849I, F880Y, R386W, P72A | P72A |

| | | | |
|---|---|---|---|
| Note that "SY43-44AA" in Table 1 represents a combination of two amino acid substitutions S43A and Y44A. | | | |

### Example 2: Effect of substitution at position R386

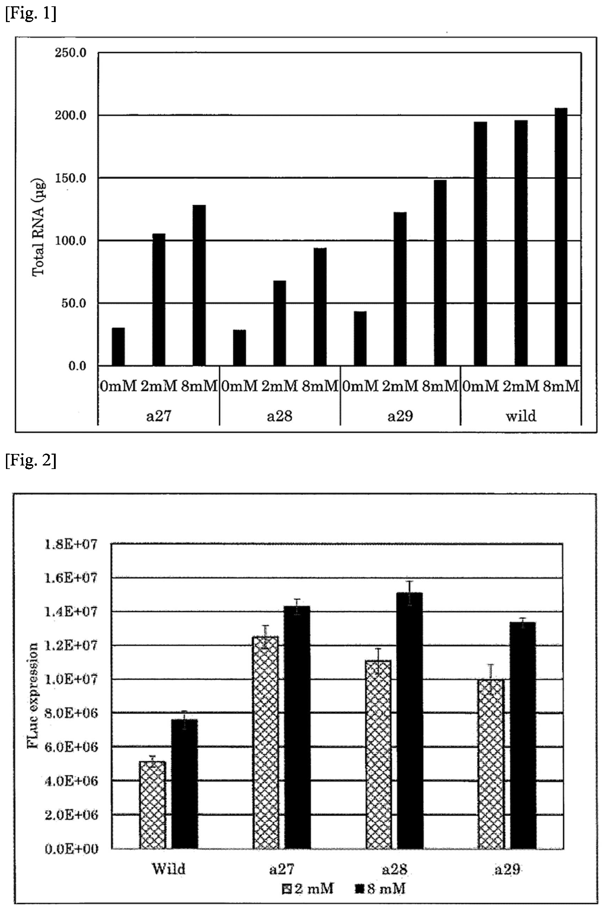

Variants of wild-type T7 RNA polymerase in which the R386 position was substituted, a27, a28, and a29 prepared in Example 1 were used to synthesize mRNA of luciferase encoded by the Positive Control Template. Three types of reaction solutions having the composition described in Experimental Method (2) were used: reaction solutions containing a cap analog [CleanCap (registered trademark) Reagent AG (3'OMe) (manufactured by Trilink)] at a final concentration of 2 mM or 8 mM, and a reaction solution containing no cap analog. As a control, the wild-type T7 RNA polymerase was used to synthesize the mRNA. A reaction mixture was incubated at 37°C for 120 minutes, and treated with DNase I to purify the RNA, after which the RNA concentration was measured. The measured total RNA amount is shown in Figure 1.

As shown in Figure 1, the three variants resulted in reduced total RNA amounts compared with the wild-type RNA polymerase. Furthermore, the total RNA amount by each variant correlated with the cap analog concentration in the reaction solution, whereas the total RNA amount by the wild-type RNA polymerase was not affected by the cap analog.

The protein expression from RNA obtained in each reaction solution was examined. To HEK293T cells (1 × 10^5 cells/well seeded and cultured for 18-24 hours) grown on a 24 well plate, a mixture of the obtained RNA (500 ng/well) and a transfection reagent (TransIT- mRNA Transfection Reagent) was added to introduce the RNA into the cells. After introduction, the cells incubated at 37°C for 24 hours, and luciferase activity in the cells was determined using ONE-Glo (registered trademark) Luciferase Assay System (manufactured by Promega) according to the procedure described in the instructions attached thereto. Results (measured values for each test) are shown in Figure 2.

As shown in Figure 2, the RNA obtained using variants a27, a28, and a29 in the reaction solutions containing the cap analog produced higher luciferase expression levels in the cells than the RNA obtained using the wild-type T7 RNA polymerase. In other words, it was suggested that the replacement of R386 in the T7 RNA polymerase with W, F, or Y resulted in more efficient synthesis of mRNA with a capped structure.

### Example 3: Evaluation of transcript RNA using T7 RNA polymerase variant

The T7 RNA polymerase variants prepared in Example 1 and the wild-type T7 RNA polymerase were subjected to evaluation tests of transcript RNA. The evaluation tests were conducted using the reaction solutions, reaction conditions, and measurement methods described in Experimental Method (2), except that two types of reaction solutions were used: one containing CleanCap (registered trademark) Reagent AG (3'OMe) at a final concentration of 8 mM, and one containing no cap analog. The total RNA amount obtained in each reaction solution and the content rate of double-stranded RNA were determined, and compared between the variants and the wild-type. Results are shown in Table 2. In the table, "T7-WT" refers to the wild-type T7 RNA polymerase.

**[Table 2]**

| | Without cap analog | | With cap analog | |
|---|---|---|---|---|
| Variant name | Total RNA amount (%) relative to T7-WT as 100% | dsRNA content rate (%) relative to T7-WT as 100% | Total RNA amount (%) relative to T7-WT as 100% | dsRNA content rate (%) relative to T7-WT as 100% |
| T7-a2_a5_a27 | 36.4 | 72 | 94.8 | 23 |
| T7-b1 | 31.7 | 130 | 81.0 | 43 |
| T7-b6 | 58.3 | 83 | 122.3 | 27 |
| T7-b7 | 69.3 | 62 | 111.5 | 15 |
| T7-b8 | 33.5 | 84 | 92.7 | 16 |
| T7-b9 | 51.6 | 134 | 127.7 | 37 |
| T7-a2_a7_a27 | 85.5 | 59 | 110.2 | 28 |
| T7-a2_a27 | 62.8 | 103 | 124.7 | 58 |

As shown in Table 2, the variant of SEQ ID NO: 6 (T7-a2_a27) produced a reduced amount of double-stranded RNA in the presence of the cap analog compared with the wild-type polymerase. The multiple variants T7-b6, b7, b8, b9, and T7-a2_a7_a27, which were created by introducing additional mutations into T7-a2_a27, further suppressed the production amount of dsRNA compared with T7-a2_a27.

### Example 5: Suppression effect of cold shock protein (CspA) on dsRNA production

CspA protein from *Escherichia coli* was prepared according to the method described in Chatterjee et al., Journal of Biochemistry, 114, pp. 663-669, 1993 and used in the following experiment. To the reaction solution described in Experimental Method (2), various concentrations of CspA and the T7 RNA polymerase contained in Takara IVTpro^{™} T7 mRNA Synthesis Kit (product number 6144, manufactured by Takara Bio Inc.) were added, and reacted at 37°C for 120 minutes. The amount of total RNA produced after the reaction and the content rate of double-stranded RNA were determined. Results are shown in Table 3.

**[Table 3]**

| CspA final conc. (ng/µl) | Total RNA (µg) | dsRNA (ng/µg Total RNA) | dsRNA (% relative to control) |
|---|---|---|---|
| 0 (control) | 174.16 | 0.20 | 100 |
| 0.8 | 177.25 | 0.17 | 85 |
| 1.6 | 179.63 | 0.14 | 72 |
| 2.4 | 172.40 | 0.15 | 74 |
| 3.2 | 174.90 | 0.12 | 60 |
| 4.0 | 172.42 | 0.12 | 58 |
| 4.8 | 161.58 | 0.12 | 61 |

As shown in Table 3, it was demonstrated that adding CspA at a final concentration of 0.8 to 4.8 µg/µl to the transcription reaction system suppressed the production of dsRNA. The total RNA levels were not significantly affected by CspA.

### Example 6: Evaluation of dsRNA production suppression by T4 Gene 32 Protein

The effect of T4 gp32 on variant T7-b7 was examined. To the reaction solution described in Experimental Method (2), various concentrations of T4 Gene 32 Protein (manufactured by NEB) and the T7 RNA polymerase contained in Takara IVTpro^{™} T7 mRNA Synthesis Kit (product number 6144, manufactured by Takara Bio Inc.) were added, and reacted at 37°C or 42°C for 120 minutes. The amount of total RNA produced after the reaction and the content rate of double-stranded RNA were determined. Results are shown in Table 4.

**[Table 4]**

| Temp (°C) | T4gp32 final Conc. (µg/µl) | Total RNA (µg) | dsRNA (ng/µg Total RNA) | dsRNA (% relative to control) |
|---|---|---|---|---|
| 37 | 0 (control) | 179.88 | 0.23 | 100 |
| | 0.2 | 172.67 | 0.20 | 86 |
| | 0.4 | 158.59 | 0.14 | 61 |
| | 0.8 | 154.91 | 0.12 | 50 |
| 42 | 0 (control) | 175.67 | 0.21 | 91 |
| | 0.2 | 169.74 | 0.15 | 65 |
| | 0.4 | 171.33 | 0.12 | 50 |
| | 0.8 | 166.07 | 0.10 | 44 |

As shown in Table 4, it was demonstrated that adding 0.2 to 0.8 µg/µl of T4 Gene 32 Protein to the reaction system suppressed the production of double-stranded RNA.

### Example 7: Evaluation of dsRNA produced by transcription of RNA of different chain lengths

Template DNAs encoding RNA of different chain lengths were prepared. The template DNAs, and the T7 RNA polymerase variant T7-b7 prepared in Example 1 and the wild-type T7 RNA polymerase were subjected to evaluation tests of transcript RNA.

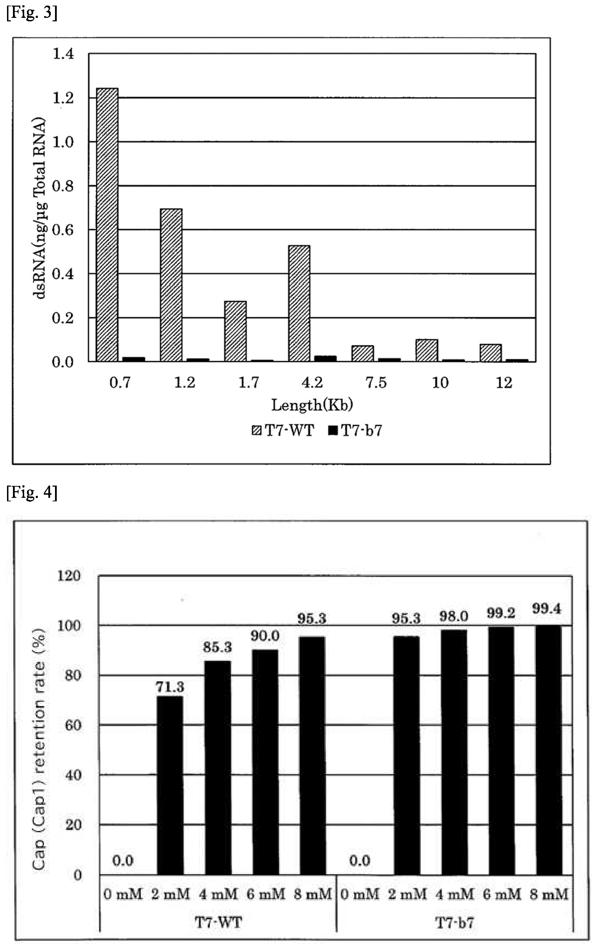

As the template DNAs encoding RNAs of different chain lengths, DNAs of seven different nucleotide sequences for generating RNAs of 0.7 kb (ZsGreen), 1.2 kb (B-actin), 1.7 kb (FLuc), 4.2 kb (Cas9), 7.5 kb (FASN), 10 kb (Lambda DNA), and 12 kb (Lambda DNA) upon transcription were prepared. Next, each plasmid DNA in which each DNA was inserted downstream of a T7 promoter was constructed. Furthermore, each plasmid was linearized using a restriction enzyme to prepare seven different DNA templates. Next, RNA transcription tests were performed using the reaction solutions, reaction conditions, and measurement methods described in Experimental Method (2), except that the linearized plasmids were used as the template DNA, and CleanCap (registered trademark) Reagent AG (3'OMe) was added to all the reaction solutions at a final concentration of 4 mM. The dsRNA content (ng) per 1 µg of total RNA was calculated from the amount of total RNA and the amount of double-stranded RNA obtained in each reaction solution, and compared between variant T7-b7 and the wild-type RNA polymerase. Results are shown in Figure 3. In the figure, "T7-WT" refers to the wild-type T7 RNA polymerase.

As shown in Figure 3, in the presence of CleanCap (registered trademark) Reagent AG (3'OMe) at a final concentration of 4 mM, variant T7-b7 produced RNA with a lower dsRNA content than the wild-type T7 RNA polymerase, regardless of the template DNA chain length.

### Example 8: Confirmation of cap analog retention rate by LC-MS

The cap retention rate of RNA prepared using the T7 RNA polymerase variant T7-b7 prepared in Example 1 and the wild-type T7 RNA polymerase was confirmed by using LC-MS (liquid chromatography-mass spectrometry). The Positive Control Template contained in Takara IVTpro (trademark) mRNA Synthesis System was used as a template to synthesize the RNA of luciferase encoded by the Positive Control Template. Five types of reaction solutions having the composition described in Experimental Method (2) were used: reaction solutions containing a cap analog [CleanCap (registered trademark) Reagent AG (3'OMe) (manufactured by Trilink)] at a final concentration of 2 mM, 4 mM, 6 mM, or 8 mM, and a reaction solution containing no cap analog. A reaction mixture was incubated at 37°C for 120 minutes, and treated with DNase I to purify the RNA. The cap (Cap1) retention rate of the purified RNA was determined by the method described in Michael Beverly et al., Anal Bioanal Chem., 2016 Jul; 408(18):5021-30 in which LC-MS analysis was used. Results are shown in Figure 4. In the figure, "T7-WT" refers to the wild-type T7 RNA polymerase.

As shown in Figure 4, the cap retention rate of RNA obtained using variant T7-b7 was higher than the cap retention rate of RNA obtained using the wild-type polymerase under all cap analog concentration conditions used in the experiment. Even when the cap analog concentration was reduced to 2 mM, using T7-b7 resulted in a cap retention rate as high as 95.3%, which was equivalent to that obtained using the wild-type polymerase in the presence of 8 mM cap analog. There was no significant difference in the amount of total RNA produced using the variant enzyme and the wild-type enzyme in the presence of the cap analog at each concentration. On the other hand, when the cap analog was not added, the amount of total RNA produced using the wild-type T7 RNA polymerase exceeded that of T7-b7. This was consistent with the results shown in Figure 1.

### INDUSTRIAL APPLICABILITY

The present invention that exhibits excellent effects in the production of high-purity mRNA is useful in a wide range of fields including genetic engineering, biology, and medicine.

### SEQUENCE LISTING FREE TEXT

SEQ ID NO1: T7-WT
SEQ ID NO2: a27
SEQ ID NO3: a28
SEQ ID NO4: a29
SEQ ID NO5: T7-a2
SEQ ID NO6: T7-a2_a27
SEQ ID NO7: T7-b2
SEQ ID NO8: T7-b3
SEQ ID NO9: T7-b4
SEQ ID NO10: T7-b5
SEQ ID NO11: T7-a2_a5_a27
SEQ ID NO12: T7-b1
SEQ ID NO13: T7-b6
SEQ ID NO14: T7-b7
SEQ ID NO15: T7-b8
SEQ ID NO16: T7-b9
SEQ ID NO17: T7-a2_a7_a27

## Claims

1. An RNA polymerase variant wherein an amino acid corresponding to arginine at position 386 in the amino acid sequence of wild-type T7 RNA polymerase set forth in SEQ ID NO: 1 is substituted by an aromatic amino acid.

2. The RNA polymerase variant according to claim 1, wherein the substitution mutation is introduced into an RNA polymerase comprising an amino acid sequence having 70% or more sequence identity with the amino acid sequence set forth in SEQ ID NO: 1.

3. The RNA polymerase variant according to claim 1 or 2, which comprises an amino acid sequence having 90% or more sequence identity with the amino acid sequence set forth in SEQ ID NO: 1.

4. The RNA polymerase variant according to any one of claims 1 to 3, wherein an amino acid corresponding to an amino acid selected from the group consisting of glutamic acid at position 48, arginine at position 50, phenylalanine at position 51, arginine at position 52, and proline at position 72 in the amino acid sequence set forth in SEQ ID NO: 1 is further substituted by an amino acid different from the original amino acid.

5. The RNA polymerase variant according to claim 4, wherein an amino acid at a position corresponding to a position selected from the group consisting of positions 48, 50, 51, 52, and 72 in the amino acid sequence set forth in SEQ ID NO: 1 is substituted by alanine.

6. The RNA polymerase variant according to any one of claims 1 to 5, wherein an amino acid corresponding to an amino acid selected from the group consisting of serine at position 430, serine at position 633, phenylalanine at position 849, and phenylalanine at position 880 in the amino acid sequence set forth in SEQ ID NO: 1 is further substituted by an amino acid different from the original amino acid.

7. The RNA polymerase variant according to claim 6, wherein an amino acid corresponding to serine at position 430 in the amino acid sequence set forth in SEQ ID NO: 1 is substituted by proline, an amino acid corresponding to serine at position 633 in the amino acid sequence set forth in SEQ ID NO: 1 is substituted by proline, an amino acid corresponding to phenylalanine at position 849 in the amino acid sequence set forth in SEQ ID NO: 1 is substituted by isoleucine, or an amino acid corresponding to phenylalanine at position 880 in the amino acid sequence set forth in SEQ ID NO: 1 is substituted by tyrosine.

8. A nucleic acid encoding the RNA polymerase variant according to any one of claims 1 to 7.

9. A vector carrying the nucleic acid according to claim 8.

10. A method for producing an RNA polymerase variant, the method comprising a step of culturing a cell into which the nucleic acid according to claim 8 has been introduced.

11. A method for producing a single-stranded RNA, the method comprising using a reaction solution containing the RNA polymerase variant according to any one of claims 1 to 7.

12. The method according to claim 11, wherein the reaction solution further contains a single-stranded DNA-binding protein, a single-stranded RNA-binding protein, or a cold shock protein.

13. The method according to claim 12, wherein the single-stranded DNA-binding protein is a T4 phage gene 32 protein.

14. The method according to claim 12, wherein the reaction solution contains a cold shock protein.

15. The method according to claim 14, wherein the cold shock protein is CspA.
